# EUROPÄISCHE PATENTSCHRIFT

(11) **EP 0 706 513 B1**
(45) Veröffentlichungstag und Bekanntmachung des Hinweises auf die Patenterteilung: **15.05.2002**
(21) Anmeldenummer: 94924713.4
(22) Anmeldetag: 02.07.1994
(51) Int. Cl.: C07D 213/75, A61K 31/44, C07C 235/56, A61K 31/165

(54) **FLUORALKOXY SUBSTITUIERTE BENZAMIDE UND IHRE VERWENDUNG ALS ZYKLISCH-NUKLEOTID PHOSPHODIESTERASE-INHIBITOREN**
FLUOROALKOXY-SUBSTITUTED BENZAMIDES AND THEIR USE AS CYCLIC NUCLEOTIDE PHOSPHODIESTERASE INHIBITORS
NOUVEAUX BENZAMIDES A SUBSTITUANTS FLUOROALCOXY ET LEUR UTILISATION COMME INHIBITEURS DE LA PHOSPHODIESTERASE NUCLEOTIDIQUE CYCLIQUE

(30) Priorität: 02.07.1993 CH 199693
(43) Veröffentlichungstag der Anmeldung: 17.04.1996
(73) Patentinhaber: Byk Gulden Lomberg Chemische Fabrik GmbH, 78467 Konstanz (DE)
(72) Erfinder: AMSCHLER, Hermann, D-78315 Radolfzell (DE); FLOCKERZI, Dieter, D-78476 Allensbach (DE); GUTTERER, Beate, D-78476 Allensbach (DE); HATZELMANN, Armin, D-78467 Konstanz (DE); SCHUDT, Christian, D-78462 Konstanz (DE); BEUME, Rolf, D-78465 Konstanz (DE); KILIAN, Ulrich, D-78479 Reichenau (DE); WOLF, Horst, P., O., D-78476 Allensbach (DE)
(86) Internationale Anmeldenummer: EP9402169
(87) Internationale Veröffentlichungsnummer: WO9501338

(56) Entgegenhaltungen:
- EP-A- 0 120 352
- EP-A- 0 163 965
- EP-A- 0 510 562
- WO-A-92/12961
- WO-A-93/25517
- WO-A-94/02465
- CHEMICAL ABSTRACTS, vol. 98, no. 23, 6. Juni 1983, Columbus, Ohio, US; abstract no. 197713n, Y. A. FIALKOV ET AL. 'Difluoromethoxy derivatives of benzoic acid and benzaldehyde' Seite 603 ; & UKR. KHIM. ZH., Bd.49, Nr.2, 1983 Seiten 187 - 189
- CHEMICAL ABSTRACTS, vol. 58, no. 9, 29. April 1963, Columbus, Ohio, US; abstract no. 8944b/c, H. HAHN 'Fluorine-containing perhaloalkyl aryl ethers' & BER., Bd.96, 1963 Seiten 48 - 55
- CHEMICAL ABSTRACTS, vol. 108, no. 15, 11. April 1988, Columbus, Ohio, US; abstract no. 131583p, S. KAZUMASA ET AL. 'Preparation of n-pyridyl-4-(benzyloxy)benzamides as cardiotonics' Seite 733 ; & JP,A,62 158 253 (KIRIN BREWERY CO.)

## Beschreibung

### Anwendungsgebiet der Erfindung

Die Erfindung betrifft neue fluoralkoxysubstituierte Benzamide, die in der pharmazeutischen Industrie zur Herstellung von Medikamenten verwendet werden.

### Bekannter technischer Hintergrund

In der internationalen Patentanmeldung WO92/12961 werden Benzamide mit PDE-hemmenden Eigenschaften beschrieben. In der internationalen Patentanmeldung WO93/25517 werden trisubstituierte Phenylderivate als selektive PDE-IV-Hemmer offenbart. In der internationalen Patentanmeldung WO94/02465 werden Inhibitoren der c-AMP Phosphodiesterase und des TNF beschrieben.

### Beschreibung der Erfindung

Es wurde nun gefunden, daß sich die nachfolgend näher beschriebenen Benzamide, die sich von den vorveröffentlichten Verbindungen insbesondere durch die Fluoralkoxysubstitution in 3-bzw. 4-Position am Benzamid unterscheiden, überraschende und besonders vorteilhafte Eigenschaften besitzen.

Gegenstand der Erfindung sind somit Verbindungen der Formel I (siehe beigefügtes Formelblatt), worin entweder
- R1: 3-7C-Cycloalkoxy, 3-7C-Cycloalkylmethoxy oder Benzyloxy und
- R2: ganz oder teilweise durch Fluor substituiertes 1-4C-Alkoxy,
oder
- R1: ganz oder teilweise durch Fluor substituiertes 1-4C-Alkoxy und
- R2: 3-7C-Cycloalkylmethoxy oder Benzyloxy bedeutet,
und
- R3: Phenyl, Pyridyl, durch R31, R32 und R33 substituiertes Phenyl oder durch R34, R35, R36 und R37 substituiertes Pyridyl bedeutet, wobei
R31 Hydroxy, Halogen, Cyano, Carboxyl, Trifluormethyl, 1-4C-Alkyl, 1-4C-Alkoxy, 1-4C-Alkoxycarbonyl, 1-4C-Alkylcarbonyl, 1-4C-Alkylcarbonyloxy, Amino, Mono-oder Di-1-4C-alkylamino oder 1-4C-Alkylcarbonylamino,
R32 Wasserstoff, Hydroxy, Halogen, Amino, Trifluormethyl, 1-4C-Alkyl oder 1-4C-Alkoxy,
R33 Wasserstoff, Halogen, 1-4C-Alkyl oder 1-4C-Alkoxy,
R34 Hydroxy, Halogen, Cyano, Carboxyl, 1-4C-Alkyl, 1-4C-Alkoxy, 1-4C-Alkoxycarbonyl oder Amino,
R35 Wasserstoff, Halogen, Amino oder 1-4C-Alkyl,
R36 Wasserstoff oder Halogen und
R37 Wasserstoff oder Halogen bedeutet,
die Salze dieser Verbindungen sowie die N-Oxide der Pyridine und deren Salze.

3-7C-Cycloalkoxy steht beispielsweise für Cyclopropyloxy, Cyclobutyloxy, Cyclopentyloxy, Cyclohexyloxy und Cycloheptyloxy, wovon Cyclopropyloxy, Cyclobutyloxy und Cyclopentyloxy bevorzugt sind.

3-7C-Cycloalkylmethoxy steht beispielsweise für Cyclopropylmethoxy, Cyclobutylmethoxy, Cyclopentylmethoxy, Cyclohexylmethoxy und Cycloheptylmethoxy, wovon Cyclopropylmethoxy, Cyclobutylmethoxy und Cyclopentylmethoxy bevorzugt sind.

Als ganz oder teilweise durch Fluor substituiertes 1-4C-Alkoxy seien beispielsweise der 1,2,2-Trifluorethoxy, der 2,2,3,3,3-Pentafluorpropoxy-, der Perfluorethoxy- und insbesondere der 1,1,2,2-Tetrafluorethoxy-, der Trifluormethoxy-, der 2,2,2-Trifluorethoxy- und der Difluormethoxyrest genannt.

Halogen im Sinne der vorliegenden Erfindung ist Brom, Chlor und Fluor.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-4C-Alkoxy steht für einen Rest, der neben dem Sauerstoffatom einen der vorstehend genannten 1-4C-Alkylreste enthält. Beispielsweise seien der Methoxy- und der Ethoxyrest genannt.

1-4C-Alkoxycarbonyl steht für eine Carbonylgruppe, an die einer der vorstehend genannten 1-4C-Alkoxyreste gebunden ist. Beispielsweise seien der Methoxycarbonyl- (CH₃O-CO-) und der Ethoxycarbonylrest (CH₃CH₂O-CO-) genannt.

1-4C-Alkylcarbonyl steht für eine Carbonylgruppe, an die einer der vorstehend genannten 1-4C-Alkylreste gebunden ist. Beispielsweise sei der Acetylrest (CH₃CO-) genannt.

1-4C-Alkylcarbonyloxyreste enthalten neben dem Sauerstoffatom einen der vorstehend genannten 1-4C-Alkylcarbonylreste. Beispielsweise sei der Acetoxyrest (CH₃CO-O-) genannt.

Als Mono- oder Di-1-4C-alkylaminoreste seien beispielsweise der Methylamino-, der Dimethylamino- und der Diethylaminorest genannt.

Als 1-4C-Alkylcarbonylaminorest sei beispielsweise der Acetylamidorest (-NH-CO-CH₃) genannt.

Als beispielhafte, durch R31, R32 und R33 substituierte Phenylreste seien die Reste 2-Acetylphenyl, 2-Aminophenyl, 2-Bromphenyl, 2-Chlorphenyl, 2,3-Dichlorphenyl, 2,4-Dichlorphenyl, 4-Diethylamino-2-methylphenyl, 4-Brom-2-trifluormethylphenyl, 2-Carboxy-5-chlorphenyl, 3,5-Dichlor-2-hydroxyphenyl, 2-Brom-4-carboxy-5-hydroxyphenyl, 2,6-Dichlorphenyl, 2,5-Dichlorphenyl, 2,4,6-Trichlorphenyl, 2,4,6-Trifluorphenyl, 2,6-Dibromphenyl, 2-Cyanphenyl, 4-Cyan-2-fluorphenyl, 2-Fluorphenyl, 2,4-Difluorphenyl, 2,6-Difluorphenyl, 2-Chlor-6-fluorphenyl, 2-Hydroxyphenyl, 2-Hydroxy-4-methoxyphenyl, 2,4-Dihydroxyphenyl, 2-Methoxyphenyl, 2,3-Dimethoxyphenyl, 2,4-Dimethoxyphenyl, 2,6-Dimethoxyphenyl, 2-Dimethylaminophenyl, 2-Methylphenyl, 2-Chlor-6-methylphenyl, 2,4-Dimethylphenyl, 2,6-Dimethylphenyl, 2,3-Dimethylphenyl, 2-Methoxycarbonylphenyl, 2-Trifluormethylphenyl, 2,6-Dichlor-4-methoxyphenyl, 2,6-Dichlor-4-cyanphenyl, 2,6-Dichlor-4-aminophenyl, 2,6-Dichlor-4-methoxycarbonylphenyl, 4-Acetylamino-2,6-dichlorphenyl und 2,6-Dichlor-4-ethoxycarbonylphenyl genannt.

Als beispielhafte, durch R34, R35, R36 und R37 substituierte Pyridylreste seien die Reste 3,5-Dichlorpyrid-4-yl, 2,6-Diaminopyrid-3-yl, 4-Aminopyrid-3-yl, 3-Methylpyrid-2-yl, 4-Methylpyrid-2-yl, 5-Hydroxypyrid-2-yl, 4-Chlorpyrid-3-yl, 3-Chlorpyrid-2-yl, 3-Chlorpyrid-4-yl, 2-Chlorpyrid-3-yl, 2,3,5,6-Tetrafluorpyrid-4-yl, 3,5-Dichlor-2,6-difluorpyrid-4-yl, 3,5-Dibrompyrid-2-yl, 3,5-Dibrompyrid-4-yl, 3,5-Dichlorpyrid-4-yl, 2,6-Dichlorpyrid-3-yl, 3,5-Dimethylpyrid-4-yl, 3-Chlor-2,5,6-trifluorpyrid-4-yl und 2,3,5-Trifluorpyrid-4-yl genannt.

Als Salze kommen für Verbindungen der Formel I - je nach Substitution- alle Säureadditionssalze aber insbesondere alle Salze mit Basen in Betracht. Besonders erwähnt seien die pharmakologisch verträglichen Salze der in der Galenik üblicherweise verwendeten anorganischen und organischen Säuren und Basen. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphtoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen vor allem auch Salze mit Basen in Betracht. Als Beispiele für basische Salze seien Lithium-, Natrium-, Kalium-, Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Hervorzuhebende Verbindungen der Formel I sind solche, in denen entweder
- R1: 3-5C-Cycloalkoxy, 3-5C-Cycloalkylmethoxy oder Benzyloxy und
- R2: ganz oder teilweise durch Fluor substutuiertes 1-4C-Alkoxy,
oder
- R1: ganz oder teilweise durch Fluor substituiertes 1-4C-Alkoxy und
- R2: 3-5C-Cycloalkylmethoxy oder Benzyloxy bedeutet
und
- R3: Phenyl, Pyridyl, durch R31, R32 und R33 substituiertes Phenyl oder durch R34, R35, R36 und R37 substituiertes Pyridyl bedeutet, wobei
R31 Halogen, Cyano, Carboxyl, 1-4C-Alkyl, 1-4C-Alkoxy oder 1-4C-Alkoxycarbonyl,
R32 Wasserstoff, Halogen, 1-4C-Alkyl oder 1-4C-Alkoxy,
R33 Wasserstoff, Halogen, 1-4C-Alkyl oder 1-4C-Alkoxy,
R34 Halogen oder 1-4C-Alkyl,
R35 Wasserstoff oder Halogen,
R36 Wasserstoff oder Halogen und
R37 Wasserstoff oder Halogen bedeutet,
die Salze dieser Verbindungen sowie die N-Oxide der Pyridine und deren Salze.

Besonderes hervorzuhebende Verbindungen der Formel I sind solche, in denen entweder
- R1: 3-5C-Cycloalkoxy, 3-5C-Cycloalkylmethoxy oder Benzyloxy und
- R2: ganz oder teilweise durch Fluor substituiertes 1-4C-Alkoxy,
oder
- R1: ganz oder teilweise durch Fluor substituiertes 1-4C-Alkoxy und
- R2: 3-5C-Cycloalkylmethoxy oder Benzxyloxy bedeutet und
- R3: 2-Bromphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2,6-Difluorphenyl, 2,6-Dichlorphenyl, 3,5-Dichlorpyrid-4-yl, 3-Methylpyrid-2-yl, 2-Chlorpyrid-3-yl, 3,5-Dibrompyrid-2-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
die Salze dieser Verbindungen sowie die N-Oxide der Pyridine und deren Salze.

Bevorzugte Verbindungen der Formel I sind solche, in denen
- R1: Difluormethoxy,
- R2: Cyclopropylmethoxy und
- R3: 2-Bromphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2,6-Difluorphenyl, 2,6-Dichlorphenyl, 3,5-Dichlorpyrid-4-yl, 3-Methylpyrid-2-yl, 2-Chlorpyrid-3-yl, 3,5-Dibrompyrid-2-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
die Salze dieser Verbindungen sowie die N-Oxide der Pyridine und deren Salze.

Weiterer Gegenstand der Erfindung ist ein Verfahren zur Herstellung der Verbindungen der Formel I und ihrer Salze, sowie der N-Oxide der Pyridine und deren Salze. Das Verfahren ist dadurch gekennzeichnet, daß man Verbindungen der Formel II (siehe beigefügtes Formelblatt), in denen R1 und R2 die oben angegebenen Bedeutungen haben und X eine geeignete Abgangsgruppe darstellt, mit Aminen R3-NH₂ umsetzt, und daß man gewünschtenfalls anschließend erhaltene Verbindungen der Formel I in ihre Salze und/oder erhaltene Pyridine in die N-Oxide und gewünschtenfalls anschließend in die Salze überführt, oder daß man gewünschtenfalls anschließend erhaltene Salze der Verbindungen der Formel I in die freien Verbindungen überführt.

Welche Abgangsgruppen X geeignet sind, ist dem Fachmann aufgrund seines Fachwissens geläufig. Beispielsweise wird von geeigneten Säurehalogeniden der Formel II (X=CI oder Br) ausgegangen. Im übrigen erfolgt die Umsetzung beispielsweise so wie in den nachfolgenden Beispielen beschrieben, oder auf eine dem Fachmann an sich vertraute Weise (z.B. so, wie in der internationalen Patentanmeldung WO 92/12961 beschrieben).

Die N-Oxidation erfolgt auf eine dem Fachmann ebenfalls vertraute Weise, z.B. mit Hilfe von m-Chlorperoxibenzoesäure in Dichlormethan bei Raumtemperatur. Welche Reaktionsbedingungen für die Durchführung des Verfahrens im einzelnen erforderlich sind, ist dem Fachmann aufgrund seines Fachwissens geläufig.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Die Verbindungen der Formel II und die Amine R3-NH2 sind bekannt, oder sie können auf bekannte Weise hergestellt werden.

Einige der nachfolgend beschriebenen Beispiele fallen nicht unter den Umfang der beanspruchten Erfindung, erleichtern aber das Verständnis der Erfindung. Bei den erfindungsgemäßen Verbindungen handelt es sich um die Beispiele 3, 5, 14 und 16.

### Beispiele

### Endprodukte

1. N-(3,5-Dichlorpyrid-4-yl)-4-difluormethoxy-3-methoxybenzamid
   6,0 g 4-Difluormethoxy-3-methoxybenzoesäure werden in 40 ml Toluol mit 19,6 g Thionylchlorid solange am Rückfluß gekocht, bis die Gasentwicklung beendet ist. Die Lösung wird im Vakuum bis zur Trockne eingedampft und der Rückstand in ca. 60 ml trockenem Tetrahydrofuran gelöst. Diese Lösung wird in eine Suspension - hergestellt aus 4,9 g 4-Amino-3,5-dichlorpyridin und 2,0 g Natriumhydrid (80-%ig in Mineralöl) in 60 ml trockenem Tetrahydrofuran - unter Rühren und Kühlen auf 15-20°C eingetropft. Nach einstündigem Rühren wird die Reaktionsmischung mit 1 N Satzsäure auf pH 2 angesäuert, die Toluol/Tetrahydrofuran-Phase abgetrennt und die wäßrige Phase noch 2 mal mit Ethylacetat extrahiert. Die vereinigten organischen Phasen werden mit gesättigter Natriumhydrogencarbonat-Lösung und Wasser gewaschen, über Natriumsulfat getrocknet und im Vakuum eingedampft. Der Rückstand wird aus Isopropanol kristallisiert. Ausbeute 5,8 g (58,6 % d.Th.) der Titelverbindung vom Fp. 170°C.
   Ausgehend von den nachfolgend beschriebenen Ausgangsverbindungen erhält man durch Umsetzung der entsprechenden Benzoesäuren mit 4-Amino-3,5-dichlorpyridin, 4-Amino-3,5-dichlor-2,6-difluorpyridin, 2,6-Dichloranilin, 2,6-Dimethylanilin, 3-Amino-2-chlorpyridin, 2-Amino-3,5-dibrompyridin, 4-Amino-2,3,5,6-tetrafluorpyridin, 2,4,6-Trifluoranilin, 2,6-Dichlor-4-ethoxycarbonylanilin, 2,6-Difluoranilin, 2-Bromanilin, 2-Chlor-6-methylanilin, 2-Methylanilin, 4-Amino-3-chlor-2,5,6-trifluorpyridin bzw. 2-Amino-3-methylpyridin analog Beispiel 1 die nachfolgend beschriebenen Endprodukte:
2. N-(3,5-Dichlorpyrid-4-yl)-3,4-bis-difluormethoxybenzamid (Fp.: 134°C)
3. N-(3,5-Dichlorpyrid-4-yl)-3-cyclobutylmethoxy-4-difluormethoxybenzamid (Fp.: 155°C)
4. N-(3,5-Dichlorpyrid-4-yl)-3-cyclopentyloxy-4-difluormethoxybenzamid (Fp.: 128,5-129°C)
5. N-(3,5-Dichlorpyrid-4-yl)-3-cyclopropylmethoxy-4-difluormethoxybenzamid (Fp.: 158°C)
6. N-(3,5-Dichlor-2,6-difluorpyrid-4-yl)-3-difluormethoxy-4-methoxybenzamid (Fp.: 218°C)
7. N-(2,6-Dichlorphenyl)-3-difluormethoxy-4-methoxybenzamid (Fp.: 164°C)
8. N-(2,6-Dimethylphenyl)-3-difluormethoxy-4-methoxybenzamid (Fp.: 164°C)
9. N-(2-Chlorpyrid-3-yl)-3-difluormathoxy-4-methoxybenzamid (Fp.: 165°C)
10. N-(3,5-Dibrompyrid-2-yl)-3-difluormethoxy-4-methoxybenzamid (Fp.: 143°C)
11. N-(3,5-Dichlorpyrid-4-yl)-3-difluormethoxy-4-methoxybenzamid (Fp.: 178°C)
12. N-(3,5-Dichlorpyrid-4-yl)-3-difluormethoxy-4-propoxybenzamid (Fp.: 159°C)
13. N-(3,5-Dichlorpyrid-4-yl)-3-ethoxy-4-difluormethoxybenzamid (Fp.: 134°C)
14. N-(3,5-Dichlorpyrid-4-yl)-4-benzyloxy-3-difluormethoxybenzamid (Fp.: 152°C)
15. N-(3,5-Dichlorpyrid-4-yl)-4-butoxy-3-difluormethoxybenzamid (Fp.: 146°C)
16. N-(3,5-Dichlorpyrid-4-yl)-4-cyclopropylmethoxy-3-difluormethoxybenzamid (Fp.: 159°C)
17. N-(3,5-Dichlorpyrid-4-yl)-4-difluormethoxy-3-(1-methylethoxy)benzamid (Fp.: 99,5°C)
18. N-(3,5-Dichlorpyrid-4-yl)-4-difluormethoxy-3-(2,2,2-trifluorethoxy)-benzamid (Fp.: 147°C)
19. N-(3,5-Dichlorpyrid-4-yl)-4-difluormethoxy-3-(2-methylpropoxy)benzamid (Fp.: 153°C)
20. N-(2,3,5,6-Tetrafluorpyrid-4-yl)-4-difluormethoxy-3-methoxybenzamid (Fp.: 146°C)
21. N-(2,4,6-Trifluorphenyl)-4-difluormethoxy-3-methoxybenzamid (Fp.: 145°C)
22. N-(2,6-Dichlor-4-ethoxycarbonylphenyl)-4-difluormethoxy-3-methoxybenzamid (Fp.: 176°C)
23. N-(2,6-Dichlorphenyl)4-difluormethoxy-3-methoxybenzamid (Fp.: 186°C)
24. N-(2,6-Difluorphenyl)-4-difluormethoxy-3-methoxybenzamid (Fp.: 139°C)
25. N-(2,6-Dimethylphenyl)-4-difluormethoxy-3-methoxybenzamid (Fp.: 143°C)
26. N-(2-Bromphenyl)-4-difluormethoxy-3-methoxybenzamid (Fp.: 121°C)
27. N-(2-Chlor-6-methylphenyl)-4-difluormethoxy-3-methoxybenzamid (Fp.: 144°C)
28. N-(2-Chlorpyrid-3-yl)-4-difluormethoxy-3-methoxybenzamid (Fp.: 137,5°C)
29. N-(2-Methylphenyl)-4-difluormethoxy-3-methoxybenzamid (Fp.: 125°C)
30. N-(3,5-Dibrompyrid-2-yl)-4-difluormethoxy-3-methoxybenzamid (Fp.: 141°C)
31. N-(3,5-Dichlor-2,6-difluorpyrid-4-yl)-4-difluormethoxy-3-methoxybenzamid (Fp.: 174°C)
32. N-(3-Chlor-2,5,6-trifluoropyrid-4-yl)-4-difluormethoxy-3-methoxybenzamid (Fp.: 141°C)
33. N-(3-Methylpyrid-2-yl)-4-difluormethoxy-3-methoxybenzamid (Fp.: 96°C)

### Ausgangsverbindungen

### A. 4-Difluormethoxy-3-methoxybenzoesäure

Zu einer Mischung von 10,0 g 4-Difluormethoxy-3-methoxybenzaldehyd, 6,5 g Amidoschwefelsäure und 50 ml Eisessig werden unter ständigem Rühren eine Lösung von 7,3 g 80-%igem Natriumchlorit in 15 ml Wasser getropft. Durch Kühlung mit Eiswasser wird die Innentemperatur zwischen 30 und 35°C gehalten. Nach beendetem Zutropfen wird noch 1 h gerührt und anschließend mit Wasser verdünnt. Die ausgefallene 4-Difluormethoxy-3-methoxybenzoesäure wird abgesaugt, mit Wasser gewaschen, im Vakuum getrocknet und aus Acetonitril/Petrolether (Sdp. 40°) 2/8 kristallisiert. Ausbeute 7,1 g; Fp. 170°C.

In analoger Weise erhält man die folgenden Benzoesäuren:
3,4-Bis-difluormethoxybenzoesäure (Fp.: 104,5°C)
3-Cyclobutylmethoxy-4-difluormethoxybenzoesäure (Fp.: 132°C)
3-Cyclopentyloxy-4-difluormethoxybenzoesäure (Fp.: 125,5°C)
3-Cyclopropylmethoxy-4-difluormethoxybenzoesäure (Fp.: 118-118,5°C)
3-Ethoxy-4-difluormethoxybenzoesäure (Fp.: 157°C)
4-Difluormethoxy-3-(1-methyl)ethoxybenzoesäure (Fp.: 93°C)
4-Difluormethoxy-3-(2,2,2-trifluorethoxy)benzoesäure (Fp.: 109°C)
3-Difluormethoxy-4-methoxybenzoesäure (Fp.: 178°C)
3-Difluormethoxy-4-propoxybenzoesäure (Fp.: 148°C)
4-Benzyloxy-3-difluormethoxybenzoesäure (Fp.: 169°C)
4-Butoxy-3-difluormethoxybenzoesäure (Fp.: 136°C)
4-Cyclopropylmethoxy-3-difluormethoxybenzoesäure (Fp.: 150°C)

### B. 4-Difluormethoxy-3-methoxybenzaldehyd

In eine Mischung von 200 g Vanillin, 6,7 g Benzyltrimethylammoniumchlorid, 314 g 50-%iger Natronlauge und 2 Liter Dioxan wird unter starkem Rühren ca. 2 h Chlordifluormethan eingeleitet. Anschließend wird die Mischung zwischen Eiswasser und Ethylacetat verteilt, die organische Phase abgetrennt, die wäßrige Phase 2 mal mit Ethylacetat ausgeführt, die vereinigten organischen Phasen über Magnesiumsulfat getrocknet und im Vakuum eingeengt. Zur Entfernung von nicht umgesetztem Vanillin wird das Öl an neutralem Kieselgel mit Toluol chromatographiert. Nach dem Eindampfen des Eluats erhält man 249 g 4-Difluormethoxy-3-methoxybenzaldehyd als Öl.

In analoger Weise erhält man die folgenden Benzaldehyde:
3-Cyclobutylmethoxy-4-difluormethoxybenzaldehyd (Fp.: 46°C)
3-Cyclopropylmethoxy-4-difluormethoxybenzaldehyd (Öl)
4-Difluormethoxy-3-(1-methyl)ethoxybenzaldehyd (Öl)
4-Difluormethoxy-3-(2,2,2-trifluorethoxy)benzaldehyd (Öl)
3,4-Bis-difluormethoxybenzaldehyd (Öl)
3-Cyclopentyloxy-4-difluormethoxybenzaldehyd (Öl)
3-Ethoxy-4-difluormethoxybenzaldehyd (Öl)
4-Difluormethoxy-3-(2-methylpropoxy)benzaldehyd (Öl)
3-Difluormethoxy-4-methoxybenzaldehyd (Fp.: 41°C)
3-Difluormethoxy-4-propoxybenzaldehyd (Öl)
4-Benzyloxy-3-difluormethoxybenzaldehyd (Fp.: 52-52,5°C)
4-Butoxy-3-difluormethoxybenzaldehyd (Öl)
4-Cyclopropylmethoxy-3-difluormethoxybenzaldehyd (Öl)

### Gewerbliche Anwendbarkeit

Die erfindungsgemäßen Verbindungen besitzen wertvolle pharmakologische Eigenschaften, die sie gewerblich verwertbar machen. Als Zyklisch-Nukleotid Phosphodiesterase (PDE) Inhibitoren (und zwar des Typs IV) eignen sie sich einerseits als Bronchialtherapeutika (zur Behandlung von Atemwegsobstruktionen aufgrund ihrer dilatierenden aber auch aufgrund ihrer atemfrequenz- bzw. atemantriebssteigernden Wirkung), andererseits jedoch vor allem zur Behandlung von Erkrankungen, insbesondere entzündlicher Natur, z.B. der Atemwege (Asthma-Prophylaxe), der Haut, des Darm, der Augen und der Gelenke, die vermittelt werden durch Mediatoren, wie Histamin, PAF (Plättchen- aktivierender Faktor), Arachidonsäure-Abkömmlinge wie Leukotriene und Prostaglandine, Zytokine, Interleukine IL-1 bis IL-12, alpha-, beta- und gamma-Interferon, Tumornekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS), Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz, oder Erkrankungen, die aufgrund der gewebsrelaxierenden Wirkung der PDE-Hemmstoffe behandelt werden können, wie beispielsweise Koliken der Nieren und der Harnleiter im Zusammenhang mit Nierensteinen.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgem können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,01 und 0,5 mg/kg. Die übliche Dosis bei systemischer Therapie liegt zwischen 0,05 und 2 mg pro Tag.

### Biologische Untersuchungen

Bei der Untersuchung der PDE IV-Hemmung auf zellulärer Ebene kommt der Aktivierung von Entzündungszellen besondere Bedeutung zu. Als Beispiel sei die FMLP (N-formyl-methionyl-leucyl-phenylalanin)-induzierte Superoxid-Produktion von neutrophilen Granulozyten genannt, die als Luminol-verstärkte Chemolumineszenz gemessen werden kann. (Mc Phail LC, Strum SL, Leone PA und Sozzani S, The neutrophil respiratory burst mechanism. In "Immunology Series" 57: 47-76, 1992; ed. Coffey RG (Marcel Decker, Inc., New York-Basel-Hong Kong)).

Substanzen, welche die Chemolumineszenz sowie die Zytokinsekretion und die Sekretion entzündungssteigernder Mediatoren an Entzündungszellen, insbesonders neutrophilen und eosinophilen Granulozyten hemmen, sind solche, welche die PDE IV hemmen. Dieses Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE IV-Hemmung durch die erfindungsgemäßen Substanzen ist damit ein zentraler Indikator für die Unterdrückung von entzündlichen Prozessen. (**Giembycz MA**, Could isoenzyme-selective phosphodiesterase inhibitors render bronchodilatory therapy redundant in the treatment of bronchial asthma?. Biochem Pharmacol 43: 2041-2051, 1992; **Torphy TJ** et al., Phosphodiesterase inhibitors: new opportunities for treatment of asthma. Thorax 46: 512-523, 1991; **Schudt C** et al., Zardaverine: a cyclic AMP PDE III/IV inhibitor. In "New Drugs for Asthma Therapy", 379-402, Birkhäuser Verlag Basel 1991; **Schudt C** et al., Influence of selective phosphodiesterase inhibitors on human neutrophil functions and levels of cAMP and Caᵢ. Naunyn-Schmiedebergs Arch Pharmacol 344: 682-690, 1991; **Nielson CP** et al., Effects of selective phosphodiesterase inhibitors on polymorphonuclear leukocyte respiratory burst. J Allergy Clin Immunol 86: 801-808, 1990; **Schade** et al., The specific type III and IV phosphodiesterase inhibitor zardaverine suppress formation of tumor necrosis factor by macrophages. European Journal of Pharmacology 230: 9-14, 1993).

### 1. Hemmung der PDE IV-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. 311, 193-198, 1980). Hierbei erfolgt im ersten Schritt die PDE-Reaktion. In einem zweiten Schritt wird das entstandene 5'-Nukleotid durch eine 5'-Nukleotidase des Schlangengiftes von ophiophagus hannah (King Cobra) zum ungeladenen Nukleosid gespalten. Im dritten Schritt wird das Nukleosid auf lonenaustauschsäulen vom verbliebenen geladenen Substrat getrennt. Die Säulen werden mit 2 ml 30 mM Ammonium formiat (pH 6,0) direkt in Minivials eluiert, in die noch 2 ml Szintillatorflüssigkeit zur Zählung gegeben wird.

Die für die erfindungsgemäßen Verbindungen ermittelten Hemmwerte ergeben sich aus der folgenden Tabelle 1, in der die Nummern der Verbindungen den Nummern der Beispiele entsprechen.

**Tabelle 1**

| Hemmung der PDE IV-Aktivität | |
|---|---|
| Verbindung | -log IC₅₀ |
| 1 | 8,64 |
| 2 | 8,42 |
| 3 | 8,74 |
| 5 | 9,18 |

### 2. Beeinflussung von Dyspnoe und Migration proinflammatorischer (immunkompetenter) Zellen aus den Blutgefäßen in das Atemwegslumen nach allergischer Reaktion von wachen Meerschweinchen

Unter dem Einfluß allergischer, inflammatorischer Reaktionen erscheinen immunkompetente Blutzellen (Leukozyten) in den Atemwegslumina. Diese pathologische Migration kann sich selbst unterhalten, d.h. zu chronischen Veränderungen führen, und spielt als Pathomechanismus von chronischer Atemwegsobstruktion (des Asthmas), aber auch von allergischer Rhinitis und/oder Conjunctivitis eine wesentliche Rolle. Die Hemmung der Migration durch Pharmaka stellt ein wichtiges therapeutisches Prinzip dar und ist ein Maß für antiallergische bzw. antiinflammatorische Wirkungen.

Die eingesetzte, tierexperimentelle Methodik zur Messung der akuten Auswirkung einer allergischen Reaktion auf die Atmung und der Zellmigration selbst folgt im wesentlichen den Beschreibungen von P.A. Hutson et al. (Am. Rev. Respir. Dis., 137, 548, 1988) und J.P. Tarayre et al. (J. Parmacol. Meth., 23, 13, 1990) sowie R. Beume et al. (Atemw. Lungenkrkh., 11, 324, 1985).

Ein definiertes Meerschweinchen-Kollektiv wird intraperitoneal gegen Ovalbumin (20 µg + 20 mg Al(OH)₃) sensibilisiert. 14 Tage später werden die Tiere in den Versuchs genommen:
- 1 h Behandlung per os,
0 h Provokation der allergischen Reaktion, thorakographisch Latenzzeitmessung bis Eintritt der Dyspnoe, Nicht-Eintritt = Schutzwirkung.
+ 1 h Behandlung per os,
+ 24 h Narkose und bronchoalveoläre Lavage: Bestimmung der Gesamtzellzahl, Auszählen des Differential-Zellbildes in der Lavage, Bestimmung des Proteingehaltes im zellfreien Überstand der Lavage.

Als Kontrolle dient je 1 Stichprobe sensibilisierter Tiere: Placebo-Behandlung + Scheinprovokation oder Placebo-Behandlung + Provokation.

Die Verbindung des Beispiel 1 (= Verbindung 1), 30 µmol/kg p.o., wirkt auf die Parameter dieser Versuchsanordnung (Relation) wie folgt:

| | **Placebo + Scheinprov.** | **Placebo + Provokation** | **Verbindung 1 + Provokation** |
|---|---|---|---|
| N, davon | 15 | 13 | 14 |
| geschützt | 15 | 2 | 8 |

| **Gesamt-Leukozyten** (x 10⁶/10 ml) | | | |
|---|---|---|---|
| Median | 2,2 (1) | 9,8 (4,5) | 2,5 (1,1) |
| Min.-Max. | 1,1-3,8 | 1,0-21,4 | 1,5-4,2 |

| **Neutrophile** Granulozyten (x 10⁶/10 ml) | | | |
|---|---|---|---|
| Median | 0,04 (1) | 0,44 (11) | 0,0 (0) |
| Min.-Max. | 0,01-0,1 | 0,01-1,1 | 0,0-0,08 |

| **Eosinophile** Granulozyten (x 10⁶/10 ml) | | | |
|---|---|---|---|
| Median | 0,8 (1) | 5,8 (7,3) | 1,2 (1,5) |
| Min.-Max. | 0,4-1,8 | 0,3-14,5 | 0,6-2,5 |

| **Makrophagen** (x 10⁶/10 ml) | | | |
|---|---|---|---|
| Median | 1,5 (1) | 3,2 (2,1) | 1,2 (0,8) |
| Min.-Max. | 0,8-2,3 | 0,7-6,2 | 0,9-2,3 |

| **Proteingehalt** (mg/10 ml) | | | |
|---|---|---|---|
| Median | 3,7 (1) | 8,8 (2,4) | 4,1 (1,1) |
| Min.-Max. | 2,3-4,9 | 3,4-22,0 | 2,6-11,0 |

Kein Tier reagiert auf die Scheinprovokation, 2 Tiere entwickeln trotz Provokation mit Ovalbumin keine Dyspnoe = spontan geschützt, unter Substanz reagieren 8 Tiere nicht = geschützt.

Die Provokation, d.h. die Auslösung einer allergischen Reaktion, erhöht den Durchtritt von Zellen und Protein deutlich, wenn auch mit großer Streuung. Verbindung 1 hemmt diese Durchtritte auf Basiswerte (Placebo + Scheinprovokation).

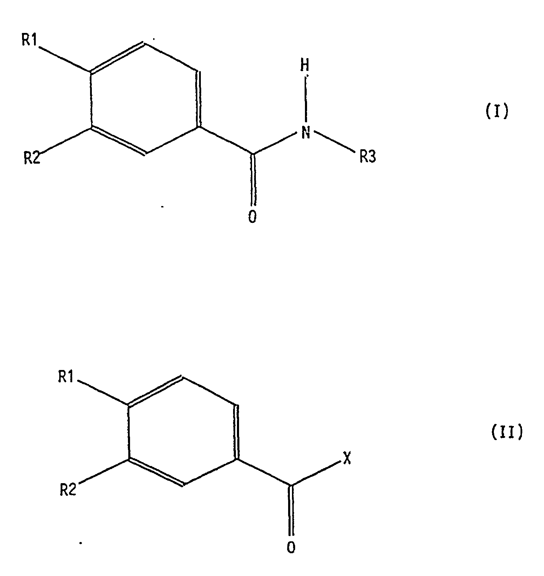

## Patentansprüche

1. Verbindungen der Formel I, 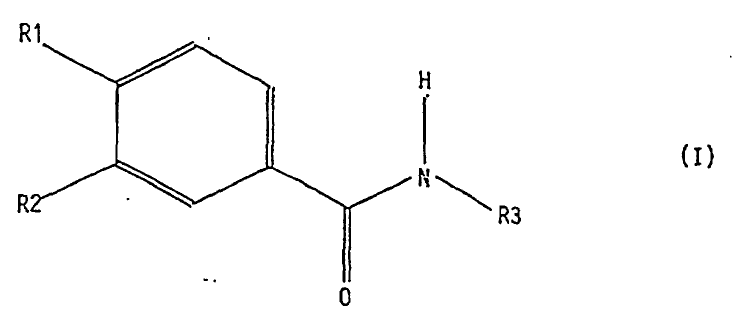 worin entweder
R1 3-7C-Cycloalkoxy, 3-7C-Cycloalkylmethoxy oder Benzyloxy und
R2 ganz oder teilweise durch Fluor substituiertes 1-4C-Alkoxy bedeutet,
oder
R1 ganz oder teilweise durch Fluor substituiertes 1-4C-Alkoxy und
R2 3-7C-Cycloalkylmethoxy oder Benzyloxy bedeutet, und
R3 Phenyl, Pyridyl, durch R31, R32 und R33 substituiertes Phenyl oder durch R34, R35, R36 und R37 substituiertes Pyridyl bedeutet, wobei
R31 Hydroxy, Halogen, Cyano, Carboxyl, Trifluormethyl, 1-4C-Alkyl, 1-4C-Alkoxy, 1-4C-Alkoxycarbonyl, 1-4C-Alkylcarbonyl, 1-4C-Alkylcarbonyloxy, Amino, Mono-oder Di-1-4C-alkylamino oder 1-4C-Alkylcarbonylamino,
R32 Wasserstoff, Hydroxy, Halogen, Amino, Trifluormethyl, 1-4C-Alkyl oder 1-4C-Alkoxy,
R33 Wasserstoff, Halogen, 1-4C-Alkyl oder 1-4C-Alkoxy,
R34 Hydroxy, Halogen, Cyano, Carboxyl, 1-4C-Alkyl, 1-4C-Alkoxy, 1-4C-Alkoxycarbonyl oder Amino,
R35 Wasserstoff, Halogen, Amino oder 1-4C-Alkyl,
R36 Wasserstoff oder Halogen und
R37 Wasserstoff oder Halogen bedeutet,
die Salze dieser Verbindungen sowie die N-Oxide der Pyridine und deren Salze.

2. Verbindungen der Formel I nach Anspruch 1,
worin
R1 3-7C-Cycloalkoxy, 3-7C-Cycloalkylmethoxy oder Benzyloxy bedeutet,
R2 ganz oder teilweise durch Fluor substituiertes 1-4C-Alkoxy bedeutet, und
R3 Phenyl, Pyridyl, durch R31, R32 und R33 substituiertes Phenyl oder durch R34, R35, R36 und R37 substituiertes Pyridyl bedeutet, wobei
R31 Hydroxy, Halogen, Cyano, Carboxyl, Trifluormethyl, 1-4C-Alkyl, 1-4C-Alkoxy, 1-4C-Alkoxycarbonyl, 1-4C-Alkylcarbonyl, 1-4C-Alkylcarbonyloxy, Amino, Mono-oder Di-1-4C-alkylamino oder 1-4C-Alkylcarbonylamino,
R32 Wasserstoff, Hydroxy, Halogen, Amino, Trifluormethyl, 1-4C-Alkyl oder 1-4C-Alkoxy,
R33 Wasserstoff, Halogen, 1-4C-Alkyl oder 1-4C-Alkoxy,
R34 Hydroxy, Halogen, Cyano, Carboxyl, 1-4C-Alkyl, 1-4C-Alkoxy, 1-4C-Alkoxycarbonyl oder Amino,
R35 Wasserstoff, Halogen, Amino oder 1-4C-Alkyl,
R36 Wasserstoff oder Halogen und
R37 Wasserstoff oder Halogen bedeutet,
die Salze dieser Verbindungen sowie die N-Oxide der Pyridine und deren Salze.

3. Verbindungen der Formel I nach Anspruch 1, worin
R1 ganz oder teilweise durch Fluor substituiertes 1-4C-Alkoxy bedeutet,
R2 3-7C-Cycloalkylmethoxy oder Benzyloxy bedeutet, und
R3 Phenyl, Pyridyl, durch R31, R32 und R33 substituiertes Phenyl oder durch R34, R35, R36 und R37 substituiertes Pyridyl bedeutet, wobei
R31 Hydroxy, Halogen, Cyano, Carboxyl, Trifluormethyl, 1-4C-Alkyl, 1-4C-Alkoxy, 1-4C-Alkoxycarbonyl, 1-4C-Alkylcarbonyl, 1-4C-Alkylcarbonyloxy, Amino, Mono-oder Di-1-4C-alkylamino oder 1-4C-Alkylcarbonylamino,
R32 Wasserstoff, Hydroxy, Halogen, Amino, Trifluormethyl, 1-4C-Alkyl oder 1-4C-Alkoxy,
R33 Wasserstoff, Halogen, 1-4C-Alkyl oder 1-4C-Alkoxy,
R34 Hydroxy, Halogen, Cyano, Carboxyl, 1-4C-Alkyl, 1-4C-Alkoxy, 1-4C-Alkoxycarbonyl oder Amino,
R35 Wasserstoff, Halogen, Amino oder 1-4C-Alkyl,
R36 Wasserstoff oder Halogen und
R37 Wasserstoff oder Halogen bedeutet,
die Salze dieser Verbindungen sowie die N-Oxide der Pyridine und deren Salze.

4. Verbindungen der Formel I nach Anspruch 1, worin
R1 3-5C-Cycloalkoxy, 3-5C-Cycloalkylmethoxy oder Benzyloxy bedeutet,
R2 ganz oder teilweise durch Fluor substituiertes 1-4C-Alkoxy bedeutet, und
R3 Phenyl, Pyridyl, durch R31, R32 und R33 substituiertes Phenyl oder durch R34, R35, R36 und R37 substituiertes Pyridyl bedeutet, wobei
R31 Halogen, Cyano, Carboxyl, 1-4C-Alkyl, 1-4C-Alkoxy oder 1-4C-Alkoxycarbonyl,
R32 Wasserstoff, Halogen, 1-4C-Alkyl oder 1-4C-Alkoxy,
R33 Wasserstoff, Halogen, 1-4C-Alkyl oder 1-4C-Alkoxy,
R34 Halogen oder 1-4C-Alkyl,
R35 Wasserstoff oder Halogen,
R36 Wasserstoff oder Halogen und
R37 Wasserstoff oder Halogen bedeutet,
die Salze dieser Verbindungen sowie die N-Oxide der Pyridine und deren Salze.

5. Verbindungen der Formel I nach Anspruch 1, worin
R1 ganz oder teilweise durch Fluor substituiertes 1-4C-Alkoxy bedeutet,
R2 3-5C-Cycloalkylmethoxy oder Benzyloxy bedeutet, und
R3 Phenyl, Pyridyl, durch R31, R32 und R33 substituiertes Phenyl oder durch R34, R35, R36 und R37 substituiertes Pyridyl bedeutet, wobei
R31 Halogen, Cyano, Carboxyl, 1-4C-Alkyl, 1-4C-Alkoxy oder 1-4C-Alkoxycarbonyl,
R32 Wasserstoff, Halogen, 1-4C-Alkyl oder 1-4C-Alkoxy,
R33 Wasserstoff, Halogen, 1-4C-Alkyl oder 1-4C-Alkoxy,
R34 Halogen oder 1-4C-Alkyl,
R35 Wasserstoff oder Halogen,
R36 Wasserstoff oder Halogen und
R37 Wasserstoff oder Halogen bedeutet,
die Salze dieser Verbindungen sowie die N-Oxide der Pyridine und deren Salze.

6. Verbindungen der Formel I nach Anspruch 1, worin
R1 3-5C-Cycloalkoxy, 3-5C-Cycloalkylmethoxy oder Benzyloxy bedeutet,
R2 ganz oder teilweise durch Fluor substituiertes 1-4C-Alkoxy bedeutet, und
R3 2-Bromphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2,6-Difluorphenyl, 2,6-Dichlorphenyl, 3,5-Dichlorpyrid-4-yl, 3-Methylpyrid-2-yl, 2-Chlorpyrid-3-yl, 3,5-Dibrompyrid-2-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
die Salze dieser Verbindungen sowie die N-Oxide der Pyridine und deren Salze.

7. Verbindungen der Formel I nach Anspruch 1, worin
R1 ganz oder teilweise durch Fluor substituiertes 1-4C-Alkoxy bedeutet,
R2 3-5C-Cycloalkylmethoxy oder Benzyloxy bedeutet, und
R3 2-Bromphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl; 2,6-Dimethylphenyl, 2,6-Difluorphenyl, 2,6-Dichlorphenyl, 3,5-Dichlorpyrid-4-yl, 3-Methylpyrid-2-yl, 2-Chlorpyrid-3-yl, 3,5-Dibrompyrid-2-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
die Salze dieser Verbindungen sowie die N-Oxide der Pyridine und deren Salze.

8. Verbindungen der Formel nach Anspruch 1, worin
R1 Difluormethoxy,
R2 Cyclopropylmethoxy und
R3 2-Bromphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2,6-Difluorphenyl, 2,6-Dichlorphenyl, 3,5-Dichlorpyrid-4-yl, 3-Methylpyrid-2-yl,2-Chlorpyrid-3-yl, 3,5-Dibrompyrid-2-yl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
die Salze dieser Verbindungen sowie die N-Oxide der Pyridine und deren Salze.

9. Verbindung der Formel I nach Anspruch 1, worin
R1 Difluormethoxy,
R2 Cyclopropylmethoxy und
R3 3,5-Dichlorpyrid-4-yl bedeutet,
die Salze dieser Verbindung sowie das N-Oxid des Pyridins und dessen Salze.

10. Verfahren zur Herstellung der Verbindungen der Formel I nach Anspruch 1 und ihrer Salze, sowie der N-Oxide der Pyridine und deren Salze, **dadurch gekennzeichnet, daß** man Verbindungen der Formel II, 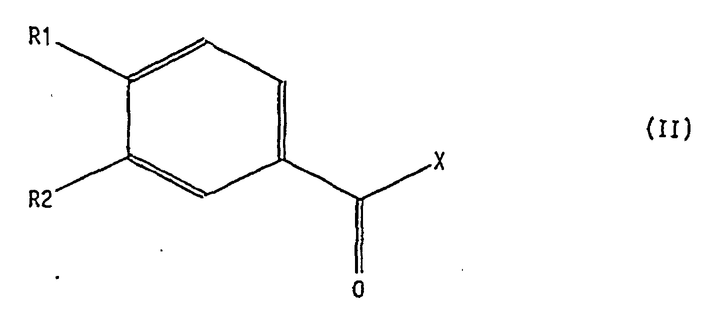 in denen R1 und R2 die in Anspruch 1 angegebenen Bedeutungen haben und X eine geeignete Abgangsgruppe darstellt, mit Aminen R3-NH₂ umsetzt, und daß man gewünschtenfalls anschließend erhaltene Verbindungen der Formel I in ihre Salze und/oder erhaltene Pyridine in die N-Oxide und gewünschtenfalls anschließend in die Salze überführt, oder daß man gewünschtenfalls anschließend erhaltene Salze der Verbindungen der Formel I in die freien Verbindungen überführt.

11. Arzneimittel enthaltend eine oder mehrere Verbindungen nach Anspruch 1 zusammen mit den üblichen pharmazeutischen Hilfsstoffen.

12. Verbindungen nach Anspruch 1 zur Anwendung bei der Behandlung von Krankheiten.

13. Verbindungen nach Anspruch 12, **dadurch gekennzeichnet, dass** es sich bei den Krankheiten um Atemwegsobstruktionen, akute oder chronische Atemwegserkrankungen, Dermatosen, Rheumatoide Arthritis, Osteoarthritis, Erkrankungen des Immunsystems, Erscheinungsformen des Schocks, Entzündungen im Magen-Darm Bereich, Herzinsuffizienz oder Koliken der Nieren und der Harnleiter im Zusammenhang mit Nierensteinen handelt.

14. Verwendung von Verbindungen nach Anspruch 1 zur Herstellung von Arzneimitteln für die Behandlung von Atemwegserkrankungen.

15. Verwendung von Verbindungen nach Anspruch 1 zur Herstellung von Arzneimitteln für die Behandlung von Dermatosen.

## Claims

1. Compounds of the formula I 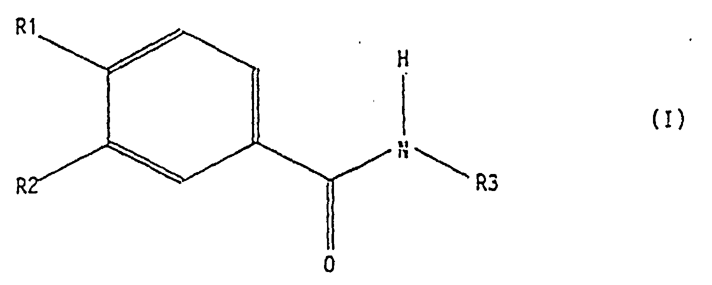 in which either
R1 is 3-7C-cycloalkoxy, 3-7C-cycloalkylmethoxy or benzyloxy, and
R2 is 1-4C-alkoxy which is completely or partially substituted by fluorine,
or
R1 is 1-4C-alkoxy which is completely or partially substituted by fluorine, and
R2 is 3-7C-cycloalkylmethoxy or benzyloxy, and
R3 is phenyl, pyridyl, phenyl which is substituted by R31, R32 and R33 or pyridyl which is substituted by R34, R35, R36 and R37, where
R31 is hydroxyl, halogen, cyano, carboxyl, tri-fluoromethyl, 1-4C-alkyl, 1-4C-alkoxy, 1-4C-alkoxycarbonyl, 1-4C-alkylcarbonyl, 1-4C-alkylcarbonyloxy, amino, mono- or di-1-4C-alkylamino or 1-4C-alkylcarbonylamino,
R32 is hydrogen, hydroxyl, halogen, amino, tri-fluoromethyl, 1-4C-alkyl or 1-4C-alkoxy,
R33 is hydrogen, halogen, 1-4C-alkyl or 1-4C-alkoxy,
R34 is hydroxyl, halogen, cyano, carboxyl, 1-4C-alkyl, 1-4C-alkoxy, 1-4C-alkoxycarbonyl or amino,
R35 is hydrogen, halogen, amino or 1-4C-alkyl,
R36 is hydrogen or halogen and
R37 is hydrogen or halogen,
the salts of these compounds, and the N-oxides of the pyridines and their salts.

2. Compounds of the formula I according to Claim 1,
in which
R1 is 3-7C-cycloalkoxy, 3-7C-cycloalkylmethoxy or benzyloxy,
R2 is 1-4C-alkoxy which is completely or partially substituted by fluorine, and
R3 is phenyl, pyridyl, phenyl which is substituted by R31, R32 and R33 or pyridyl which is substituted by R34, R35, R36 and R37, where
R31 is hydroxyl, halogen, cyano, carboxyl, tri-fluoromethyl, 1-4C-alkyl, 1-4C-alkoxy, 1-4C-alkoxycarbonyl, 1-4C-alkylcarbonyl, 1-4C-alkylcarbonyloxy, amino, mono- or di-1-4C-alkylamino or 1-4C-alkylcarbonylamino,
R32 is hydrogen, hydroxyl, halogen, amino, tri-fluoromethyl, 1-4C-alkyl or 1-4C-alkoxy,
R33 is hydrogen, halogen, 1-4C-alkyl or 1-4C-alkoxy,
R34 is hydroxyl, halogen, cyano, carboxyl, 1-4C-alkyl, 1-4C-alkoxy, 1-4C-alkoxycarbonyl or amino,
R35 is hydrogen, halogen, amino or 1-4C-alkyl,
R36 is hydrogen or halogen and
R37 is hydrogen or halogen,
the salts of these compounds, and the N-oxides of the pyridines and their salts.

3. Compounds of the formula I according to Claim 1,
in which
R1 is 1-4C-alkoxy which is completely or partially substituted by fluorine,
R2 is 3-7C-cycloalkylmethoxy or benzyloxy, and
R3 is phenyl, pyridyl, phenyl which is substituted by R31, R32 and R33 or pyridyl which is substituted by R34, R35, R36 and R37, where
R31 is hydroxyl, halogen, cyano, carboxyl, tri-fluoromethyl, 1-4C-alkyl, 1-4C-alkoxy, 1-4C-alkoxycarbonyl, 1-4C-alkylcarbonyl, 1-4C-alkylcarbonyloxy, amino, mono- or di-1-4C-alkylamino or 1-4C-alkylcarbonylamino,
R32 is hydrogen, hydroxyl, halogen, amino, tri-fluoromethyl, 1-4C-alkyl or 1-4C-alkoxy,
R33 is hydrogen, halogen, 1-4C-alkyl or 1-4C-alkoxy,
R34 is hydroxyl, halogen, cyano, carboxyl, 1-4C-alkyl, 1-4C-alkoxy, 1-4C-alkoxycarbonyl or amino,
R35 is hydrogen, halogen, amino or 1-4C-alkyl,
R36 is hydrogen or halogen and
R37 is hydrogen or halogen,
the salts of these compounds, and the N-oxides of the pyridines and their salts.

4. Compounds of the formula I according to Claim 1, in which
R1 is 3-5C-cycloalkoxy, 3-5C-cycloalkylmethoxy or benzyloxy,
R2 is 1-4C-alkoxy which is completely or partially substituted by fluorine, and
R3 is phenyl, pyridyl, phenyl substituted by R31, R32 and R33 or pyridyl substituted by R34, R35, R36 and R37, where
R31 is halogen, cyano, carboxyl, 1-4C-alkyl, 1-4C-alkoxy or 1-4C-alkoxycarbonyl,
R32 is hydrogen, halogen, 1-4C-alkyl or 1-4C-alkoxy,
R33 is hydrogen, halogen, 1-4C-alkyl or 1-4C-alkoxy,
R34 is halogen or 1-4C-alkyl,
R35 is hydrogen or halogen,
R36 is hydrogen or halogen and
R37 is hydrogen or halogen,
the salts of these compounds, and the N-oxides of the pyridines and their salts.

5. Compounds of the formula I according to Claim 1, in which
R1 is 1-4C-alkoxy which is completely or partially substituted by fluorine,
R2 is 3-5C-cycloalkylmethoxy or benzyloxy, and
R3 is phenyl, pyridyl, phenyl substituted by R31, R32 and R33 or pyridyl substituted by R34, R35, R36 and R37, where
R31 is halogen, cyano, carboxyl, 1-4C-alkyl, 1-4C-alkoxy or 1-4C-alkoxycarbonyl,
R32 is hydrogen, halogen, 1-4C-alkyl or 1-4C-alkoxy,
R33 is hydrogen, halogen, 1-4C-alkyl or 1-4C-alkoxy,
R34 is halogen or 1-4C-alkyl,
R35 is hydrogen or halogen,
R36 is hydrogen or halogen and
R37 is hydrogen or halogen,
the salts of these compounds, and the N-oxides of the pyridines and their salts.

6. Compounds of the formula I according to Claim 1, in which
R1 is 3-5C-cycloalkoxy, 3-5C-cycloalkylmethoxy or benzyloxy,
R2 is 1-4C-alkoxy which is completely or partially substituted by fluorine, and
R3 is 2-bromophenyl, 2,6-dichloro-4-ethoxycarbonyl-phenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichloro-phenyl, 3,5-dichloropyrid-4-yl, 3-methylpyrid-2-yl, 2-chloropyrid-3-yl, 3,5-dibromopyrid-2-yl, 2,3,5,6-tetrafluoropyrid-4-yl, 3-chloro-2,5,6-trifluoro-pyrid-4-yl, 3,5-dichloro-2,6-difluoropyrid-4-yl or 2,6-dichloropyrid-3-yl,
the salts of these compounds, and the N-oxides of the pyridines and their salts.

7. Compounds of the formula I according to Claim 1, in which
R1 is 1-4C-alkoxy which is completely or partially substituted by fluorine,
R2 is 3-5C-cycloalkylmethoxy or benzyloxy, and
R3 is 2-bromophenyl, 2,6-dichloro-4-ethoxycarbonyl-phenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichloro-phenyl, 3,5-dichloropyrid-4-yl, 3-methylpyrid-2-yl, 2-chloropyrid-3-yl, 3,5-dibromopyrid-2-yl, 2,3,5,6-tetrafluoropyrid-4-yl, 3-chloro-2,5,6-trifluoro-pyrid-4-yl, 3,5-dichloro-2,6-difluoropyrid-4-yl or 2,6-dichloropyrid-3-yl.
the salts of these compounds, and the N-oxides of the pyridines and their salts.

8. Compounds of the formula I according to Claim 1, in which
R1 is difluoromethoxy,
R2 is cyclopropylmethoxy and
R3 is 2-bromophenyl, 2,6-dichloro-4-ethoxycarbonyl-phenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichloro-phenyl, 3,5-dichloropyrid-4-yl, 3-methylpyrid-2-yl, 2-chloropyrid-3-yl, 3,5-dibromopyrid-2-yl, 2,3,5,6-tetrafluoropyrid-4-yl, 3-chloro-2,5,6-trifluoro-pyrid-4-yl, 3,5-dichloro-2,6-difluoropyrid-4-yl or 2,6-dichloropyrid-3-yl,
the salts of these compounds, and the N-oxides of the pyridines and their salts.

9. Compound of the formula I according to Claim 1, in which
R1 is difluoromethoxy,
R2 is cyclopropylmethoxy and
R3 is 3,5-dichloropyrid-4-yl,
the salts of this compound, and the N-oxide of the pyridine and its salts.

10. Process for the preparation of the compounds of the formula I according to Claim 1 and their salts, and the N-oxides of the pyridines and their salts, **characterized in that** compounds of the formula II 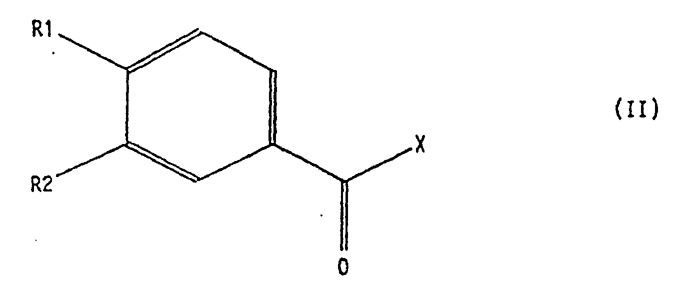 in which R1 and R2 have the meanings indicated in Claim 1 and X is a suitable leaving group, are reacted with amines R3-NH₂, and **in that**, if desired, compounds of the formula I obtained are then converted into their salts and/or pyridines obtained are converted into the N-oxides and, if desired, then into the salts, or **in that**, if desired, salts of the compounds of the formula I obtained are then converted into the free compounds.

11. Medicaments containing one or more compounds according to Claim 1, together with customary pharmaceutical auxiliaries.

12. Compounds according to Claim 1 for use in the treatment of illnesses.

13. Compounds according to Claim 12, **characterized in that** the illnesses are airway obstructions, acute and chronic airway disorders, dermatoses, rheumatoid arthritis, osteoarthritis, disorders of the immune system, types of shock, inflammations in the gastrointestinal region, cardiac insuffi-ciency, or colics of the kidneys and of the ureters in connection with kidney stones.

14. Use of compounds according to Claim 1 for the production of medicaments for the treatment of airway disorders.

15. Use of compounds according to Claim 1 for the production of medicaments for the treatment of dermatoses.

## Revendications

1. Composés de formule I 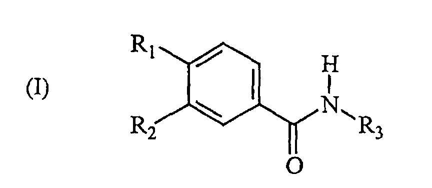 dans laquelle
soit R₁ représente un groupe cycloalcoxy en C₃₋₇, (cycloalkyle en C₃₋₇)-méthoxy ou benzyloxy et R₂ représente un groupe alkoxy en C₁₋₄ partiellement ou complètement fluoré,
soit R₁ est un groupe alcoxy en C₁₋₄ partiellement ou complètement fluoré et R₂ représente un groupe (cycloalkyle en C₃₋₇)-méthoxy ou benzyloxy, et
R₃ représente un groupe phényle, pyridyle, phényle substitué par R₃₁, R₃₂ et R₃₃, ou pyridyle substitué par R₃₄, R₃₅, R₃₆ et R₃₇,
où
R₃₁ représente un groupe hydroxy, halogéno, cyano, carboxyle, trifluorométhyle, alkyle en C₁₋₄, alcoxy en C₁₋₄, (alcoxy en C₁₋₄)-carbonyle, (alkyle en C₁₋₄)-carbonyle, (alkyle en C₁₋₄)-carbonyloxy, amino, mono- ou di(alkyle en C₁₋₄)-amino ou (alkyle en C₁₋₄)-carbonylamino,
R₃₂ représente un atome d'hydrogène ou d'halogène, un groupe hydroxy, amino, trifluorométhyle, alkyle en C₁₋₄ ou alcoxy en C₁₋₄,
R₃₃ représente un atome d'hydrogène ou d'halogène, un groupe alkyle en C₁₋₄ ou alcoxy en C₁₋₄,
R₃₄ représente un atome d'halogène ou un groupe hydroxy, cyano, carboxyle, alkyle en C₁₋₄, alcoxy en C₁₋₄, (alcoxy en C₁₋₄)-carbonyle ou amino,
R₃₅ représente un atome d'hydrogène ou d'halogène, un groupe amino ou alkyle en C₁₋₄,
R₃₆ représente un atome d'hydrogène ou d'halogène, et
R₃₇ représente un atome d'hydrogène ou d'halogène,
les sels de-ces composés ainsi que les N-oxydes des pyridines et leurs sels.

2. Composés de formule (I) selon la revendication 1, dans laquelle
R₁ représente un groupe cycloalcoxy en C₃₋₇, (cycloalkyle en C₃₋₇)-méthoxy ou benzyloxy,
R₂ représente un groupe alcoxy en C₁₋₄, partiellement ou complètement fluoré, et
R₃ représente un groupe phényle, pyridyle, phényle substitué par R₃₁, R₃₂ et R₃₃, ou pyridyle substitué par R₃₄, R₃₅, R₃₆ et R₃₇,
où
R₃₁ représente un groupe hydroxy, halogéno, cyano, carboxyle, trifluorométhyle, alkyle en C₁₋₄, alcoxy en C₁₋₄, (alcoxy en C₁₋₄)-carbonyle, (alkyle en C₁₋₄)-carbonyle, (alkyle en C₁₋₄)-carbonyloxy, amino, mono- ou di(alkyle en C₁₋₄)-amino ou (alkyle en C₁₋₄)-carbonylamino,
R₃₂ représente un atome d'hydrogène ou d'halogène, un groupe hydroxy, amino, trifluorométhyle, alkyle en C₁₋₄ ou alcoxy en C₁₋₄,
R₃₃ représente un atome d'hydrogène ou d'halogène, un groupe alkyle en C₁₋₄ ou alcoxy en C₁₋₄,
R₃₄ représente un atome d'halogène ou un groupe hydroxy, cyano, carboxyle, alkyle en C₁₋₄, alcoxy en C₁₋₄, (alcoxy en C₁₋₄)-carbonyle ou amino,
R₃₅ représente un atome d'hydrogène ou d'halogène, un groupe amino ou alkyle en C₁₋₄,
R₃₆ représente un atome d'hydrogène ou d'halogène, et
R₃₇ représente un atome d'hydrogène ou d'halogène,
les sels de ces composés ainsi que les N-oxydes des pyridines et leurs sels.

3. Composés de formule (I) selon la revendication 1, dans laquelle
R₁ représente un groupe alcoxy en C₁₋₄, partiellement ou complètement fluoré,
R₂ représente un groupe (cycloalkyle en C₃₋₇)-méthoxy ou benzyloxy, et
R₃ représente un groupe phényle, pyridyle, phényle substitué par R₃₁, R₃₂ et R₃₃, ou pyridyle substitué par R₃₄, R₃₅, R₃₆ et R₃₇,
où
R₃₁ représente un groupe hydroxy, halogéno, cyano, carboxyle, trifluorométhyle, alkyle en C₁₋₄, alcoxy en C₁₋₄, (alcoxy en C₁₋₄)-carbonyle, (alkyle en C₁₋₄)-carbonyle, (alkyle en C₁₋₄)-carbonyloxy, amino, mono- ou di(alkyle en C₁₋₄)-amino ou (alkyle en C₁₋₄)-carbonylamino,
R₃₂ représente un atome d'hydrogène ou d'halogène, un groupe hydroxy, amino, trifluorométhyle, alkyle en C₁₋₄ ou alcoxy en C₁₋₄,
R₃₃ représente un atome d'hydrogène ou d'halogène, un groupe alkyle en C₁₋₄ ou alcoxy en C₁₋₄,
R₃₄ représente un atome d'halogène ou un groupe hydroxy, cyano, carboxyle, alkyle en C₁₋₄, alcoxy en C₁₋₄, (alcoxy en C₁₋₄)-carbonyle ou amino,
R₃₅ représente un atome d'hydrogène ou d'halogène, un groupe amino ou alkyle en C₁₋₄,
R₃₆ représente un atome d'hydrogène ou d'halogène, et
R₃₇ représente un atome d'hydrogène ou d'halogène,
les sels de ces composés ainsi que les N-oxydes des pyridines et leurs sels.

4. Composés de formule (I) selon la revendication 1, dans laquelle
R₁ représente un groupe cycloalcoxy en C₃₋₅, (cycloalkyle en C₃₋₅)-méthoxy ou benzyloxy et
R₂ représente un groupe alcoxy en C₁₋₄, partiellement ou complètement fluoré, et
R₃ représente un groupe phényle, pyridyle, phényle substitué par R₃₁, R₃₂ et R₃₃, ou pyridyle substitué par R₃₄, R₃₅, R₃₆ et R₃₇,
où
R₃₁ représente un atome d'halogène, un groupe cyano, carboxyle, alkyle en C₁₋₄, alcoxy en C₁₋₄ ou (alcoxy en C₁₋₄)-carbonyle,
R₃₂ représente un atome d'hydrogène ou d'halogène, ou un groupe alkyle en C₁₋₄ ou alcoxy en C₁₋₄,
R₃₃ représente un atome d'hydrogène ou d'halogène, ou un groupe alkyle en C₁₋₄ ou alcoxy en C₁₋₄,
R₃₄ représente un atome d'halogène ou un groupe alkyle en C₁₋₄,
R₃₅ représente un atome d'hydrogène ou d'halogène,
R₃₆ représente un atome d'hydrogène ou d'halogène, et
R₃₇ représente un atome d'hydrogène ou d'halogène,
les sels de ces composés ainsi que les N-oxydes des pyridines et leurs sels.

5. Composés de formule (I) selon la revendication 1, dans laquelle
R₁ représente un groupe alcoxy en C₁₋₄, partiellement ou complètement fluoré,
R₂ représente un groupe (cycloalkyle en C₃₋₅)-méthoxy ou benzyloxy, et
R₃ représente un groupe phényle, pyridyle, phényle substitué par R₃₁, R₃₂ et R₃₃, ou pyridyle substitué par R₃₄, R₃₅, R₃₆ et R₃₇,
où
R₃₁ représente un atome d'halogène, un groupe cyano, carboxyle, alkyle en C₁₋₄, alcoxy en C₁₋₄ ou (alcoxy en C₁₋₄)-carbonyle,
R₃₂ représente un atome d'hydrogène ou d'halogène, un groupe alkyle en C₁₋₄ ou alcoxy en C₁₋₄,
R₃₃ représente un atome d'hydrogène ou d'halogène, un groupe alkyle en C₁₋₄ ou alcoxy en C₁₋₄,
R₃₄ représente un atome d'halogène ou un groupe alkyle en C₁₋₄,
R₃₅ représente un atome d'hydrogène ou d'halogène,
R₃₆ représente un atome d'hydrogène ou d'halogène, et
R₃₇ représente un atome d'hydrogène ou d'halogène,
les sels de ces composés ainsi que les N-oxydes des pyridines et leurs sels.

6. Composés de formule (I) selon la revendication 1, dans laquelle
R₁ représente un groupe cycloalcoxy en C₃₋₅, (cycloalkyle en C₃₋₅)-méthoxy ou benzyloxy,
R₂ représente un groupe alcoxy en C₁₋₄, partiellement ou complètement fluoré, et
R₃ représente un groupe 2-bromophényle, 2,6-dichloro-4-éthoxycarbonylphényle, 2,6-diméthoxyphényle, 4-cyano-2-fluorophényle, 2,4,6-trifluorophényle, 2-chloro-6-méthylphényle, 2,6-diméthylphényle, 2,6-difluorophényle, 2,6-dichlorophényle, 3,5-dichloropyrid-4-yle, 3-méthylpyrid-2-yle, 2-chloropyrid-3-yle, 3,5-dibromopyrid-2-yle, 2,3,5,6-tétrafluoropyrid-4-yle, 3-chloro-2,5,6-trifluoropyrid-4-yle, 3,5-dichloro-2,6-difluoropyrid-4-yle ou 2,6-dichloropyrid-3-yle,
les sels de ces composés ainsi que les N-oxydes des pyridines et leurs sels.

7. Composés de formule (I) selon la revendication 1, dans laquelle
R₁ représente un groupe alcoxy en C₁₋₄, partiellement ou complètement fluoré,
R₂ représente un groupe (cycloalkyle en C₃₋₅)-méthoxy ou benzyloxy, et
R₃ représente un groupe 2-bromophényle, 2,6-dichloro-4-éthoxycarbonylphényle, 2,6-diméthoxyphényle, 4-cyano-2-fluorophényle, 2,4,6-trifluorophényle, 2-chloro-6-méthylphényle, 2,6-diméthylphényle, 2,6-difluorophényle, 2,6-dichlorophényle, 3,5-dichloropyrid-4-yle, 3-méthylpyrid-2-yl, 2-chloropyrid-3-yle, 3,5-dibromopyrid-2-yle, 2,3,5,6-tétrafluoropyrid-4-yle, 3-chloro-2,5,6-trifluoropyrid-4-yle, 3,5-dichloro-2,6-difluoropyrid-4-yle ou 2,6-dichloropyrid-3-yle,
les sels de ces composés ainsi que les N-oxydes des pyridines et leurs sels.

8. Composés de formule (I) selon la revendication 1, dans laquelle
R₁ représente un groupe difluorométhoxy,
R₂ représente un groupe cyclopropylméthoxy, et
R₃ représente un groupe 2-bromophényle, 2,6-dichloro-4-éthoxycarbonylphényle, 2,6-diméthoxyphényle, 4-cyano-2-fluorophényle, 2,4,6-trifluorophényle, 2-chloro-6-méthylphényle, 2,6-diméthylphényle, 2,6-difluorophényle, 2,6-dichlorophényle, 3,5-dichloropyrid-4-yle, 3-méthylpyrid-2-yle, 2-chloropyrid-3-yle, 3,5-dibromopyrid-2-yle, 2,3,5,6-tétrafluoropyrid-4-yle, 3-chloro-2,5,6-trifluoropyrid-4-yle, 3,5-dichloro-2,6-difluoropyrid-4-yle ou 2,6-dichloropyrid-3-yle,
les sels de ces composés ainsi que les N-oxydes des pyridines et leurs sels.

9. Composés de formule (I) selon la revendication 1, dans laquelle
R₁ représente un groupe difluorométhoxy,
R₂ représente un groupe cyclopropylméthoxy, et
R₃ représente un groupe 3,5-dichloropyrid-4-yle,
les sels de ces composés ainsi que le N-oxyde de la pyridine et ses sels.

10. Procédé de préparation de composés de formule (I) selon la revendication 1 et de leurs sels, ainsi que des N-oxydes des pyridines et de leurs sels, **caractérisé par le fait que** l'on fait réagir des composés de formule (II) 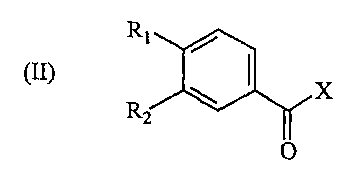 dans laquelle R₁ et R₂ ont la signification indiquée dans la revendication 1 et X représente un groupe partant approprié, avec des amines de formule R₃-NH₂, et que l'on convertit ensuite éventuellement les composés de formule (I) obtenus en leurs sels et/ou les pyridines obtenues en les N-oxydes correspondants et ensuite éventuellement en leurs sels, ou que l'on convertit éventuellement les sels obtenus en les composés libres de formule (I).

11. Médicament contenant un ou plusieurs composés selon la revendication 1 avec des excipients pharmaceutiques usuels.

12. Composés selon la revendication 1 pour le traitement de maladies.

13. Composés selon la revendication 12, **caractérisés par le fait que** les maladies englobent les obstructions des voies respiratoires, les maladies aigües ou chroniques des voies respiratoires, les dermatoses, l'arthrite rhumatoïde, l'arthrite osseuse, les maladies du système immunitaire, les différentes formes de chocs, les maladies inflammatoires du tractus gastro-intestinal, l'insuffisance cardiaque ou les coliques néphritiques et coliques des urètres associées à des calculs rénaux.

14. Utilisation de composés selon la revendication 1 pour la fabrication de médicaments destinés au traitement de maladies des voies respiratoires.

15. Utilisation de composés selon la revendication 1 pour la fabrication de médicaments destinés au traitement de dermatoses.
